# EUROPEAN PATENT SPECIFICATION

(11) **EP 1 713 439 B1**
(45) Date of publication and mention of the grant of the patent: **01.07.2009**
(21) Application number: 04804078.6
(22) Date of filing: 20.12.2004
(51) Int. Cl.: A61K 9/00, A61K 31/192, A61K 47/18, A61P 11/04, A61P 29/00, A61K 31/196

(54) **PHARMACEUTICAL PREPARATION FOR THE ORAL CAVITY**
PHARMAZEUTISCHES PRÄPARAT FÜR DIE MUNDHÖHLE
PREPARATION PHARMACEUTIQUE DESTINEE A LA CAVITE ORALE

(30) Priority: 13.02.2004 IT MI20040235
(43) Date of publication of application: 25.10.2006
(73) Proprietor: THERAPICON SRL, I-20146 Milan (IT)
(72) Inventor: VERONESI, Paulo, Alberto, 1-20144 Milano (IT)
(74) Representative: Rickard, David John
(86) International application number: PCT/EP2004/014478
(87) International publication number: WO 2005/079747

(56) References cited:
- EP-A- 0 726 075
- WO-A-98/52540
- WO-A-02/094246
- WO-A-03/094905
- GB-A- 1 072 359
- GB-A- 1 494 558
- GB-A- 1 540 483
- US-A- 5 183 829
- US-A- 5 889 057
- HOLZER P ET AL: "Estimation of acute flurbiprofen and ketoprofen toxicity in rat gastric mucosa at therapy-relevant doses" INFLAMMATION RESEARCH, vol. 50, no. 12, December 2001 (2001-12), pages 602-608, XP001204998 ISSN: 1023-3830

## Description

The invention relates generally to a pharmaceutical preparation for the oral cavity consisting of an aqueous solution, buffered to a physiological pH, provided with antiinflammatory and analgesic activity, particularly suitable for spraying into the oral cavity by means of a suitable dosing pump.

For around a decade now, the incidence of generalised inflammatory conditions of the throat, mouth and gums has been on the increase, especially during the winter. These very troublesome conditions are not generally attributable to a specific cause, but may arise due to various external factors, such as for example sudden changes in ambient temperature, irritant or toxic substances contained in the air or in polluted environments, direct or indirect inhalation of cigarette smoke, or internal factors, such as for example slight infections with viruses, echoviruses, macro viruses or bacteria or, as frequently occurs, due to the simultaneous presence of one or more of these irritants. The resultant clinical picture is thus highly complex, with inflammation and pain predominating among the many symptoms. Since it is consequently not possible to combat each of these various causes individually with a specific, targeted treatment, the only possible therapeutic strategy is to eliminate the troublesome symptoms of these conditions as effectively as possible, primarily by countering the inflammation or the congestion of the throat, mouth and gums, while simultaneously also alleviating or eliminating the troublesome pain.

The products usable to treat this complex clinical picture which are currently commercially available may in general terms be divided into two categories.

The first of these consists of a range of products based on natural substances or extracts, such as propolis, mixtures of honey and wild rose, eugenol and others. The second category, on the other hand, comprises medicinal preparations containing one or more pharmaceutical active ingredients which must combine efficacy with an optimum safety and tolerability profile. These medicinal preparations are generally classified by the European health authorities as "self -medication products", which the patient may accordingly request on his/her own initiative or after consulting a doctor, pharmacist or any other health professional or in response to advertising messages.

These pharmaceutical products, although subject to prior approval as medicines by the health regulatory authorities (since they contain one or more active ingredients) and thus frequently being sold only in pharmacies (the specific legislation may vary from country to country), may be freely sold directly to any patient requesting them without there being any need to submit a doctor's prescription. This explains the alternative names for these medicines which are also known as "freely sold products" or "over-the-counter products".

Taking due account of the above, a medicinal product for self medication to be used as an antiinflammatory and analgesic for spraying into/onto the mouth, throat and gums must necessarily meet various ideal requirements, including:
(a) satisfactory antiinflammatory and analgesic activity, both for reducing congestion and for alleviating the associated pain; the active ingredient must furthermore be homogeneously dissolved in the solution so that it can be sprayed uniformly into the oral cavity;
(b) the solution must be pharmaceutically stable and the active and auxiliary ingredients must accordingly not react with one another;
(c) the solution must be biologically acceptable to the oral mucosa, and thus neither excessively acidic, so as not to attack the dentine, nor excessively basic, so as not to exacerbate the irritation;
(d) provision of a mild disinfectant action to protect the mouth and pharynx from any bacterial and viral attack;
(e) have a preservative action to protect the solution from bacterial contamination and proliferation during production and subsequent use;
(f) be organoleptically acceptable since it is intended for an organ which is particularly delicate and sensitive to unpleasant flavours and odours.

An ideal aqueous solution must remain stable for a certain period of time, being clear and transparent without precipitates and contaminants. It will be necessary to avoid certain incompatibilities, such as using parabens with a pH greater than 8.0, introducing a highly reactive inorganic substance, such as for example potassium bicarbonate, into the composition, using edetic acid and some of the salts thereof which attack the calcium of the dentine ("Handbook of Pharmaceutical Excipients", 4th edition, 2003, American Pharmaceutical Association, page 226, paragraph 14, Safety) or using unstable colorants in order to avoid loss of colour during ageing and so on.

At present, there is no pharmaceutical composition available which is capable of combining all the ideal features listed above. Indeed, the formulations which may be found in the literature or those already on the market (trade names are deliberately not stated so as not to give rise to any unjustified accusation of unfair competition) lack the majority of the properties listed above.

The new generation nonsteroidal antiinflammatories, such as for example COX-2 inhibitors (celecoxib, rofecoxib and others), cannot be used topically due their mechanism of action. Other first generation nonsteroidal antiinflammatory drugs (NSAIDs), on the other hand, cannot be used due to the high concentration which is required (ibuprofen, tiaprofenic acid), or due to their known instability in water (acetylsalicylic acid), or also due to their sparing solubility (piroxicam, tenoxicam). Still others are known to have sensitising potential (diflunisal, zomepirac), which makes topical use thereof inadvisable.

Of the remaining active ingredients, some (naproxen and etodolac) exhibit a predominant antiinflammatory activity and inadequate analgesic activity, while others conversely exhibit a predominant analgesic activity (ketorolac) and little antiinflammatory activity. Some products already on the market occasionally exhibit a pH of greater than 8.0 and are thus not physiologically compatible with the mucosa and furthermore result in harmful dysmicrobism of the oral cavity's saprophytic flora. The physiological pH of the mouth is in fact between 6.7 and 7.5.

Holzer et al, 2001, "Estimation of acute flurbiprofen and ketoprofen toxicity in rat gastric mucosa at therapy-relevant doses", Inflammation Research, vol.50, no.12, 2001, pages 602-608 discloses solutions of racemic flurbiprofen and of its enantiomers in saline. US 5,183,829 (Caldwell et al) 2 February 1993 discloses oral solutions comprising diclofenac or flurbiprofen in the form of their tromethamine salt.

Given that no pharmaceutical composition which is described in the literature or is commercially available is capable of meeting the requirements listed above, there is accordingly an urgent need to fill this gap with a pharmaceutical preparation which combines the features listed above.

### General

After various studies and experimental trials, a pharmaceutical preparation combining said features has now surprisingly been found. According to one embodiment of the present invention there is provided an oromucosal solution comprising:
(a) the nonsteroidal antiinflammatory drug (NSAID) flurbiprofen which has analgesic activity, in a quantity of from 1.5 mg/ml to 8.0 mg/ml;
(b) a biologically compatible buffer consisting essentially of an organic amine selected from at least one of D-glucamine, meglumine, trometamol (tris buffer) and a mixture thereof, in a quantity suitable for buffering the pH of the preparation within the range specified below;
(c) a pH within a range from 6.5 to 8.0 and preferably from 7.0 to 7.5; and
(d) pharmaceutical grade water.

According to another embodiment the present invention also relates to use of an oromucosal solution for the manufacture of an anti-inflammatory agent for treating the mouth, throat and/or gums, comprising:
the nonsteroidal anti-inflammatory drug (NSAID) flurbiprofen which has analgesic activity, in a quantity of from 1.5 mg/ml to 8.0 mg/ml;
a biologically compatible buffering organic amine provided with a free or monosubstituted amino group or a mixture thereof, in a quantity suitable for buffering the pH of the preparation within the range specified below;
a pH within a range of from 6.5 to 8.0; and
pharmaceutical grade water;
wherein the biologically compatible buffering organic amine is D-glucamine, meglumine, trometamol (tris buffer) or a mixture thereof.

The solution buffered in this manner may furthermore contain:
(e) a mild disinfectant
(f) one or more preservatives
(g) other auxiliary ingredients.

The invention may also relate to the pharmaceutical dosage form based on the solution defined above. Said solution may furthermore be distributed in a container with volume ranging from 10 to 100 ml.

The invention may also relate to the complete packaged form of the solution defined above, which consists of a container, which encloses the buffered solution, provided with a dosing pump and a suitable distributor ' for spraying the solution directly into the oral cavity.

The invention may also relate to a process for the production of the oromucosal solution of the invention, said process comprising the following sequence of operations;
i) dissolving preservative(s) in a solution;
   ii) dissolving the flurbiprofen in water or a water/ethyl alcohol mixture and buffering with the organic amine to the specified pH value;
   iii) adding any auxiliary ingredients to the solution of step i), and mixing the solution of step i) with the solution of flurbiprofen and organic amino from step ii);
   iv) making up to volume (or weight) with water, if necessary, adjusting the pH to the prescribed value with addition of organic amine.

The buffered solution may be apportioned into a container, which is sealed with the dosing pump: the suitable distributor is fitted onto the pump and the system is then packaged into its box with the patient information leaflet.

The invention will now be illustrated in greater detail in the following description.

The invention provides an oromucosal solution which comprises:
(A) the nonsteroidal antiinflammatory drug (NSAID) flurbiprofen in the quantity of from 1.5 mg/ml to 8.0 mg/ml
Flurbiprofen exhibits an high therapeutic index. Flurbiprofen may be employed as a racemate (or racemic mixture) or as one of its enantiomers, namely (R) - (-) flurbiprofen or (S) - (+) flurbiprofen, and more particularly (R) - (-) flurbiprofen. The flurbiprofen is used alone in the solution in a range of concentration within which the optimum concentration has been determined for the type of indication, as shown in Table 1 below:

**Table 1**

| NSAID | Minimum concentration in mg/ml (% wt./vol.) | Maximum concentration in mg/ml (% wt./vol.) | Optimum concentration in mg/ml (% wt./vol.) |
|---|---|---|---|
| Flurbiprofen | 1.5 (0.15%) | 8.0 (0.8%) | 2.5 (0.25%) |

(B) a biologically compatible organic amine with pronounced buffering properties, present alone or as a mixture, with the buffering amino group in free or partially substituted form, used in a sufficient quantity to maintain the pH of the solution within a specified range close to the physiological pH of the oral cavity. The selected buffering organic amine consists of D-glucamine, meglumine, or trometamol (tris buffer). Meglumine in particular, having a methyl monosubstituted amino group and thus a weaker buffering action, as has also been described in the literature (Merck Index 13th ed. / meglumine 1.0% = pH 10.5 and trometamol 0.1% = pH 10.1), is more readily suitable to obtain the desired pH. Trometamol, on the other hand, is also highly advisable, being described in the classic, most reliable textbooks of chemical pharmacology as the only "non-toxic amine" to act as a "biological buffer".
The desired buffering action is generally obtained at a concentration which varies for each buffering organic amine and is stated in Table 2 below.

**Table 2**

| Buffering substance | Minimum concentration In mg/ml (% wt./vol.) | Maximum concentration in mg/ml (% wt./vol.) |
|---|---|---|
| Glucamine | 0.35 (0.035%) | 1.12 (0.112%) |
| Meglumine | 0.40 (0.04%) | 2.4 (0.24%) |
| Trometamol (tris buffer) | 0.10 (0.01%) | 0.75 (0.075%) |

(C) the pH of the solution is within a range between 6.5 and 8.0, preferably between 7.0 and 7.5.
This pH value is accordingly obtained by buffering the specified quantity of the selected NSAID with the (mono- or disubstituted) buffering organic amine in the quantity required to obtain a biocompatible pH as close as possible to the physiological pH of the mouth, which lies between 6.7 and 7.5. This pH range is furthermore particularly suitable for avoiding any modification of the physiological balance of the saprophytic bacterial flora of the oral cavity.
(D) pharmaceutical grade water, such as purified or twice-distilled water, of the quality specified in the usual pharmacopoeias.
Preferably the oromucosal solution of the invention provides a buffered solution which exhibits further improvements in terms of its pharmaceutical, technical and organoleptic properties.
The present invention preferably provides a buffered solution which is also suitable for combatting superficial infective conditions arising from bacterial or viral infections, there is also an objective requirement to provide:
(E) a mild surface disinfectant which is biologically and pharmaceutically compatible with topical use and is selected from among those conventionally used for similar topical indications and applications in a quantity which is familiar to the person skilled in the art. This substance must furthermore be chemically compatible with the other ingredients of the solution and with the dispensing system used. The disinfectant which is typically selected consists of cetylpyridinium chloride or of glycyrrhizic acid or the ammonium or dipotassium salts thereof, the antibacterial and antiviral properties of which have already been thoroughly described in the literature. The disinfectant substance is present alone in the solution, in a sufficient quantity to exert a specific antibacterial and antiviral action. Besides, glycyrrhizic acid, or the ammonium or dipotassium salt thereof, also exhibits a considerable sweet flavour approx. 50 times more powerful than sucrose.
The mild disinfectant selected is used alone in the buffered solution in a variable quantity in a range within which the optimum concentration has also been determined, as shown in Table 3 below:

**Table 3**

| Mild disinfectant | Minimum concentration in mg/ml (% wt./vol.) | Maximum concentration in mg/ml (% wt./vol.) | Optimum concentration in mg/ml (% wt./vol.) |
|---|---|---|---|
| Cetylpyridinium chloride | 1.0 (0.01 %) | 6.0 (0.6%) | 5.0 (0.5%) |
| Glycyrrhizic acid or salts thereof | 0.8 (0.08%) | 1.2 (0.12%) | 1.0 (0.1%) |

The buffered solution of the invention may furthermore generally be packaged for preservation, distribution and subsequent use in a multidose container, equipped with a suitable pressure dosing pump which makes it possible to spray the solution uniformly into/onto the throat, mouth and gums. In this case, however, there is a real risk that, due to the reduction in internal pressure arising from repeated use of the pump, contaminated air will enter the container from outside causing accidental contamination or the proliferation of bacterial colonies in the solution itself.
Thus, unless a more advanced pump is used, which is already commercially available, although at higher cost, and is equipped with a suitable filtration system which sterilises the air entering the container to compensate the reduction in internal pressure, the buffered solution should preferably also comprise:
(F) a preservative substance, or a mixture thereof, which is selected from among those conventionally used and in the quantity familiar to the person skilled in the art, in order to achieve sufficient microbiological control of the solution, and is moreover compatible with the topical mode of administration and also from the chemical standpoint not only with the other ingredients of the solution, but also with the components of the multidose system used. The typical preservatives selected comprise not only conventional parabens, such as methyl p-hydroxybenzoate or propyl p-hydroxybenzoate, each of which alone or in combination, but in particular also disodium calcium edetate (i.e. not the simply disodium salt which is capable of attacking the calcium in tooth enamel), or finally sodium benzoate. The selected preservative is used in the buffered solution at the appropriate concentration to prevent bacterial contamination and proliferation, as shown in Table 4 below:

**Table 4**

| Preservative substance | Minimum concentration in mg/ml (% wt./vol.) | Maximum concentration in mg/ml (% wt./vol.) |
|---|---|---|
| Methyl p-hydroxybenzoate | 0.25 (0.025%) | 1.15 (0.115%) |
| Propyl p-hydroxybenzoate | 0.03 (0.003%) | 0.15 (0.015%) |
| Disodium calcium edetate | 0.1 (0.01%) | 1.0 (0.1%) |
| Sodium benzoate | 0.2 (0.02%) | 5.0 (0.5%) |

Finally, in order to improve the final technical, pharmaceutical and organoleptic properties of the buffered solution, bearing in mind that flavour is a non-negligible factor in a product which is intended to be sprayed into the oral cavity, preferably the pharmaceutical preparation of the invention is improved from the technical and organoleptic standpoint by the addition of other auxiliary ingredients, as indicated below.
(G) The nature, the quality and the concentration of each individual auxiliary ingredient varies from case to case depending on the starting buffered solution and on the final properties of the preparation which it is desired to obtain.
With regard to the quality of an individual auxiliary ingredient, a person skilled in the art will certainly be capable of selecting that which complies with the quality specifications stated in the specific monograph published in one of the main pharmacopoeias (Eur. Ph., USP, JP, FU, BP). In the absence of a specific monograph, the person skilled in the art will be able to select the auxiliary ingredient with properties which comply as well as possible with those stated in specialist publications, such as for example "Remington: The Science and Practice of Pharmacy", 20th Edition, editors A.R. Gennaro et al., University of the Sciences in Philadelphia College or "Handbook of Pharmaceutical Excipients", 4th Edition, 2003, American Pharmaceutical Association.
The following Examples provide purely indicative examples of specific auxiliary ingredients and the associated optimum concentrations for each buffered solution illustrated in the Examples themselves.
The preferred auxiliary ingredients which are selected and thus also the concentration thereof are accordingly not binding for each buffered solution and do not limit the invention, it being possible to replace each of them suitably with another similar ingredient while still obtaining a result which is comparable overall with that of the invention itself.
Nevertheless, with regard to the quality and quantity thereof stated in the Examples, these ingredients are the result of careful optimisation which was not carried out casually but also involved an inventive step.
The preferred auxiliary ingredients for the following Examples are stated below:
- glycerol (viscosity agent)
- sorbitol, xylitol (sweetening agent)
- ethyl alcohol (fluidising agent)
- castor oil 40 polyethoxylate (thickening agent)
- saccharin sodium, acesulfame potassium (sweeteners)
- mint essence, natural mint flavour, natural peach flavour (natural essences or flavours)
- patent blue V-E131, E-124 (colours)

Preferably the invention provides an oromucosal solution wherein xylitol is used as a noncariogenic sweetening agent. It will be appreciated that xylitol is not utilized by microorganisms and does not promote dental plaque with the associated cariogenic effects. However, xylitol exerts certain bacteriostatic and bactericidal affects, particularly against common spoilage organisms, thus enhancing the stability and freshness of the composition. Moreover, it will be appreciated that a solution according to a preferred embodiment of the invention containing xylitol is also not contraindicated in diabetic or carbohydrate-controlled diets.

A solution according to one embodiment of the invention is prepared in the above-stated sequence using the methods and machinery conventionally used in the pharmaceutical sector, but this is neither mandatory nor does it limit the invention itself. Indeed, adjustments remain possible with regard to the specific formulation used, the overall volume of the batch to be prepared, while nevertheless obtaining a result which is comparable overall with that of the invention itself.

The solution may generally be packaged for preservation, distribution, sale and use in a suitable container provided with a dosing pump with an associated distributor, in such a manner that it may readily be sprayed directly into/onto the mouth, throat and gums. In particular, the solution is preferably packaged in a multidose container equipped with a pressure operating pump, fitted with a dispensing erogator (of variable type and shape) which enables uniform spraying of the solution within the oral cavity.

In general, the volume of solution sprayed for each dose varies as a function of the concentration of the active ingredient, but for the formulations of the Examples, the ideal volume to be sprayed for each dose ranges from 100 to 300 microlitres, with an amount of 200 microlitres preferably being sprayed for each unit dose.

The oromucosal solution of the invention may be useful for the topical treatment of inflammatory conditions of the mouth, throat and gums with accompanying pain and, where the composition also contains a mild disinfectant, also for combatting the condition brought about by the bacterial and viral component which is often associated therewith. The preparation may also be useful in reducing the inflammation/congestion and associated pain of the mucosa of the oral cavity.

Examples of typical buffered solutions of the invention are presented below in tabular form in order to make the individual details more readily discernible.

These Examples are provided with the aim of better illustrating the invention and thus do not constitute any limitation of the invention itself.

### EXAMPLES 1 TO 3

| **INGREDIENT** | **TYPE** | | **EXAMPLES 1 TO 3 (mg/ml)** | | |
|---|---|---|---|---|---|
| | | | 1 | 2 | 3 |
| Flurbiprofen | A | mg | 2.50 | 2.50 | 2.50 |
| Glucamine to make up to pH(C) | B | mg | --- | --- | --- |
| Meglumine to make up to pH(C) | B | mg | + 2.10 | +2.15 | +0.70 |
| Trometamol to make up to pH(C) | B | mg | --- | --- | +0.40 |
| pH | C | | 7.10 | 7.30 | 7.20 |
| Cetylpyridinium chloride | E | mg | --- | --- | --- |
| Glycyrrhizic acid | E | mg | --- | --- | --- |
| Methyl p-hydroxybenzoate | F | mg | 1.00 | --- | 1.00 |
| Propyl p-hydroxybenzoate | F | mg | 0.20 | --- | 0.20 |
| Disodium calcium edetate | F | mg | --- | 0.50 | --- |
| Sodium benzoate | F | mg | --- | --- | --- |
| Glycerol | G | mg | 100.00 | 100.00 | 100.00 |
| Sorbitol | G | mg | 70.00 | 70.00 | 70.00 |
| Xylitol | G | mg | --- | --- | --- |
| Ethyl alcohol (96%) | G | mg | 100.00 | 100.00 | 100.00 |
| Hydrogenated castor oil 40 polyethoxylate | G | mg | 24.00 | 24.00 | 24.00 |
| Saccharin sodium | G | mg | 1.50 | 1.50 | 1.50 |
| Acesulfame potassium | G | mg | --- | --- | --- |
| Mint essence | G | mg | 6.00 | 6.00 | 6.00 |
| Natural mint flavour | G | mg | --- | --- | --- |
| Natural peach flavour | G | mg | --- | --- | --- |
| Patent blue V-E131 | G | mg | --- | 0.006 | --- |
| Colour E124 | G | mg | --- | --- | --- |
| Purified water up to volume | D | ml | 1.00 | 1.00 | 1.00 |

| | | | | | |
|---|---|---|---|---|---|
| (A) = Active ingredient (B) = Buffering organic amine (C) =pH (D) =Water (Pharmaceutical grade) (E) = Disinfectant (F) = Preservative (G) =Auxiliary ingredient | | | | | |

The following compositions are prepared as described in the method of the subsequent Example.

### EXAMPLES 4 & 5

| **INGREDIENT** | **TYPE** | | **EXAMPLES 4 & 5 (mg/ml)** | |
|---|---|---|---|---|
| | | | **4** | **5** |
| Flurbiprofen | A | mg | 2.50 | 2.50 |
| Glucamine to make up to pH (C) | B | mg | --- | + 1.00 |
| Meglumine to make up to pH (C) | B | mg | + 2.30 | --- |
| Trometamol to make up to pH (C) pH | B | mg | --- | --- |
| | C | | 7.00 | 7.50 |
| Cetylpyridinium chloride | E | mg | 5.00 | --- |
| Glycyrrhizic acid | E | mg | --- | 1.00 |
| Methyl p-hydroxybenzoate | F | mg | 1.00 | --- |
| Propyl p-hydroxybenzoate | F | mg | 0.20 | --- |
| Disodium calcium edetate | F | mg | --- | 0.50 |
| Sodium benzoate | F | mg | --- | --- |
| Glycerol | G | mg | 100.00 | 100.00 |
| Sorbitol | G | mg | 70.00 | 70.00 |
| Xylitol | G | mg | --- | --- |
| Ethyl alcohol (96%) | G | mg | 100.00 | 100.00 |
| Hydrogenated castor oil 40 polyethoxylate | G | mg | 24.00 | 24.00 |
| Saccharin sodium | G | mg | 1.50 | 1.50 |
| Acesulfame potassium | G | mg | --- | --- |
| Mint essence | G | mg | 6.00 | 6.00 |
| Natural mint flavour | G | mg | --- | --- |
| Natural peach flavour | G | mg | --- | --- |
| Patent blue V-E131 | G | mg | --- | 0.008 |
| Colour E124 | G | mg | --- | --- |
| Purified water up to volume | D | ml | 1.00 | 1.00 |

| | | | | |
|---|---|---|---|---|
| (A) =Active Ingredient (B) =Buffering organic amine (C) =pH (D) =Water (pharmaceutical grade) (E) =Disinfectant (F) =Preservative (G) auxiliary ingredient | | | | |

The following compositions are prepared as described in the method of the subsequent Example.

### EXAMPLES 6 & 7

| **INGREDIENT** | **TYPE** | | **EXAMPLES 6 & 7** **(mg/ml)** | |
|---|---|---|---|---|
| | | | **6** | **7** |
| Flurbiprofen | A | mg | 2.50 | 2.50 |
| Glucamine to make up to pH (C) | B | mg | --- | --- |
| Meglumine to make up to pH (C) | B | mg | + 2.10 | --- |
| Trometamol to make up to pH (C) | B | mg | --- | + 0.70 |
| pH | C | | 7.10 | 7.40 |
| Cetylpyridinium chloride | E | mg | --- | --- |
| Glycyrrhizic acid | E | mg | --- | --- |
| Methyl p-hydroxybenzoate | F | mg | 1.00 | 1.00 |
| Propyl p-hydroxybenzoate | F | mg | 0.20 | 0.20 |
| Disodium calcium edetate | F | mg | --- | --- |
| Sodium benzoate | F | mg | --- | --- |
| Glycerol | G | mg | 100.00 | 100.00 |
| Sorbitol | G | mq | --- | --- |
| Xylitol | G | mg | 70.00 | 70.00 |
| Ethyl alcohol (96%) | G | mg | 100.00 | 104.00 |
| Hydrogenated castor oil 40 polyethoxylate | G | mg | 24.00 | 24.00 |
| Saccharin sodium | G | mg | 1.50 | 1.50 |
| Acesulfame potassium | G | mg | --- | --- |
| Mint essence | G | mg | 6.00 | 6.00 |
| Natural mint flavour | G | mg | --- | --- |
| natural peach flavour | G | mg | --- | --- |
| Patent blue V-E131 | G | mg | --- | 0.006 |
| Colour E124 | G | mg | --- | --- |
| purified water up to volume | D | ml | 1.00 | 1.00 |

| | | | | |
|---|---|---|---|---|
| (A) =Active ingredient (B) =Buffering organic amine (C) =pH (D) =Water (pharmaceutical grade) (E) =Disinfectant (F) =Preservative (G) =Auxillary ingredient | | | | |

The following compositions are prepared as described in the method of the subsequent Example.

### EXAMPLE 8

### Preparation of 2000 vials containing 15 ml of solution for spraying according to the composition of Example 1.

Production
for 2000 vials containing 15 ml of solution for spraying:

| Ingredient | 15 ml Vial | | Total | |
|---|---|---|---|---|
| Flurbiprofen | 37.50 | mg | 75.00 | g |
| Meglumine to make up to pH(C) | + 31.50 | mg | + 63.00 | g |
| pH | 7.10 | | 7.10 | |
| Methyl p-hydroxybenzoate | 15.00 | mg | 30.00 | g |
| Propyl p-hydroxybenzoate | 3.00 | mg | 6.00 | g |
| Glycerol | 1.50 | g | 3.00 | kg |
| Sorbitol | 1.05 | g | 2.10 | kg |
| Ethyl alcohol (96%) | 1.50 | g | 3.10 | kg |
| Hydrogenated castor oil 40 polyethoxylate | 360.00 | mg | 720.00 | g |
| Saccharin sodium | 22.50 | mg | 45.00 | g |
| Mint essence | 90.00 | mg | 180.00 | g |
| Purified water up to volume | 15.00 | ml | 30.00 | 1 |

### Phase 1 - Solution A

20 litres of purified water are placed in a suitable stainless steel dissolver and adjusted to approx. 80 °C. Completely dissolve 30.0 g of methyl p-hydroxybenzoate and 6.0 g of propyl p-hydroxybenzoate. Cool the solution to ambient temperature (25°C).

### Phase 2 - Solution B

3 litres of water and 3.0 kg of 96% ethyl alcohol are mixed in a suitable stainless steel container at approx. 30°C. Then add 75.0 g of flurbiprofen and buffer to pH 7.1 with meglumine (approx. 63 g).

### Phase 3 - Solution C

While continuously stirring solution A, add the other ingredients: 3.0 kg of glycerol, 2.1 kg of sorbitol, 720.0 g of hydrogenated castor oil 40 polyethoxylate, 45.0 g of saccharin sodium and 180.0 g of mint essence. Stir until dissolution is complete.

### Phase 4 - Buffered solution

Adjust the volume to 30 litres by adding purified water and check the pH, if necessary, buffer the pH to the desired value of 7.1 by adding meglumine.

The buffered solution is then apportioned into the vials which are sealed with the dosing pump equipped with a dispensing erogator. The system is then packaged in a suitable box. In this manner, 1865 vials each of 15 ml are obtained.

## Claims

1. An oromucosal solution comprising:
the nonsteroidal antiinflammatory drug (NSAID) flurbiprofen, which has analgesic activity, in a quantity of from 1.5 mg/ml to 8.0 mg/ml;
a biologically compatible buffer consisting essentially of an organic amine selected from at least one D-glucamine, meglumine, trometamol (tris buffer) and a mixture thereof, in a quantity suitable for buffering the pH of the preparation within the range specified below;
a pH within a range from 6.5 to 8.0; and
pharmaceutical grade water.

2. An oromucosal solution according to claim 1, wherein the flurbiprofen is in the form of a racemate or one of its enantiomers selected from R-(-) flurbiprofen and S-(+) flurbiprofen.

3. An oromucosal solution according to any one of claims 1 or 2, which comprises flurbiprofen in a quantity of about 2.5 mg/ml.

4. An oromucosal solution according to any one of the preceding claims, which has a pH of from 7.0 to 7.5.

5. An oromucosal solution according to any one of the preceding claims, which comprises D-glucamine in a quantity of from 0.35 mg/ml to 1.12 mg/ml; meglumine in a quantity of from 0.40 mg/ml to 2.4 mg/ml; and/or trometamol in a quantity of from 0.10 mg/ml to 0.75 mg/ml.

6. An oromucosal solution according to any one of the preceding claims, which comprises the buffer in a quantity suitable for buffering the pH of the solution within the range of from 7.0 to 7.5.

7. An oromucosal solution according to any one of the preceding claims, which further comprises:
a mild disinfectant; and/or
one or more preservatives;
wherein:
the mild disinfectant comprises at least one of (i) cetylpyridinium chloride, optionally in a quantity of from 1.0.mg/ml to 6.0 mg/ml, optimally of about 5.0 mg/ml, and (ii) glycyrrhizic acid or a salt thereof, optionally in a quantity of from 0.8 mg/ml to 1.2 mg/ml, optimally of about 1.0 mg/ml;
the preservative comprises at least one of (i) methyl p-hydroxybenzoate, optionally in a quantity of from 0.25 mg/ml to 1.15 mg/ml, (ii) propyl p-hydroxybenzoate, optionally in a quantity of from 0.03 mg/ml to 0.15 mg/ml, (iii) disodium calcium edetate, optionally in a quantity of from 0.1 mg/ml to 1.0 mg/ml, and (iv) sodium benzoate, optionally in a quantity of from 0.2 mg/ml to 5.0 mg/ml.

8. An oromucosal solution according to any one of the preceding claims, which further comprises at least one further ingredient selected from a viscosity agent, a sweetening agent, a fluidising agent, a thickening agent, a colouring agent and a natural essence of flavouring agent.

9. An oromucosal solution according to claim 8, wherein the further ingredient is selected from at least one of glycerol, sorbitol, xylitol, ethyl alcohol, castor oil 40 polyethoxylate, saccharin sodium, acesulfame potassium, mint essence, natural mint flavour, natural peach flavour and patent blue V-E131, E-124.

10. An oromucosal solution according to any one of claims 1 to 8 further comprising xylitol.

11. An oromucosal solution according to any one of the preceding claims in the form of a mouthwash for spraying, preferably with a dispensed volume for each unit dose of from 100 microlitres (0.1 ml) to 300 microlitres (0.3 ml), preferably of about 200 microlitres (0.2 ml).

12. An oromucosal solution according to any one of the preceding claims, wherein the buffer is D-glucamine, meglumine or a mixture thereof.

13. A packaged oromucosal solution containing the pharmaceutical preparation defined in any one of claims 1 to 12, equipped with a dosing pump.

14. A process for the production of the pharmaceutical preparation defined in any one of claims 1 to 12, which comprises
i) dissolving preservative(s) in a solution;
ii) dissolving the flurbiprofen in water or a water/ethyl alcohol mixture and buffering with the organic amine to the specified pH value;
iii) adding any auxiliary ingredients to the solution of step i), and mixing the solution of step i) with the solution of flurbiprofen and organic amine from step ii);
iv) making up to volume (or weight) with water, if necessary, adjusting the pH to the prescribed value with addition of organic amine.

15. Use of an oromucosal solution for the manufacture of an anti-inflammatory agent for treating the mouth, throat and/or gums, wherein the pharmaceutical composition is in the form of an aqueous solution comprising:
the nonsteroidal anti-inflammatory drug (NSAID) flurbiprofen which has analgesic activity in a quantity of from 1.5 mg/ml to 8.0 mg/ml;
a biologically compatible buffering organic amine provided with a free or monosubstituted amino group or a mixture thereof, in a quantity suitable for buffering the pH of the preparation within the range specified below;
a pH within a range of from 6.5 to 8.0; and
pharmaceutical grade water; and
the biologically compatible buffering organic amine is D-glucamine, meglumine, trometamol (tris buffer) or a mixture thereof.

## Patentansprüche

1. Oromukosale Lösung, die umfasst:
das nichtsteroidale Antirheumatikum (NSAR) Flurbiprofen, welches analgetisch wirkt, in einer Menge von 1,5 mg/ml bis 8,0 mg/ml;
einen biologisch kompatiblen Puffer, der im Wesentlichen aus einem organischen Amin besteht, welches aus D-Glukamin und/oder Meglumin und/oder Trometamol (Tris-Puffer) und/oder einer Mischung davon in einer zum Puffern des pH-Werts des Präparats in dem unten angegebenen Bereich geeigneten Menge ausgewählt ist;
einen pH-Wert in einem Bereich von 6,5 bis 8,0; und Reinstwasser.

2. Oromukosale Lösung gemäß Anspruch 1, wobei das Flurbiprofen die Form eines Razemats oder eines seiner Enantiomere hat, die aus R-(-)-Flurbiprofen und S-(+)-Flurbiprofen ausgewählt sind.

3. Oromukosale Lösung gemäß Anspruch 1 oder 2, welche Flurbiprofen in einer Menge von ungefähr 2,5 mg/ml umfasst.

4. Oromukosale Lösung gemäß einem der vorangehenden Ansprüche, welche einen pH-Wert von 7,0 bis 7,5 aufweist.

5. Oromukosale Lösung gemäß einem der vorangehenden Ansprüche, welche D-Glukamin in einer Menge von 0,35 mg/ml bis 1,12 mg/ml umfasst; Meglumin in einer Menge von 0,40 mg/ml bis 2,4 mg/ml; und/oder Trometamol in einer Menge von 0,10 mg/ml bis 0,75 mg/ml.

6. Oromukosale Lösung gemäß einem der vorangehenden Ansprüche, welche den Puffer in einer zum Puffern des pH-Werts der Lösung im Bereich von 7,0 bis 7,5 geeigneten Menge umfasst.

7. Oromukosale Lösung gemäß einem der vorangehenden Ansprüche, welche ferner umfasst:
ein mildes Desinfektionsmittel; und/oder
einen oder mehrere Konservierungsstoffe;
wobei:
das milde Desinfektionsmittel mindestens (i) Cetylpyridiniumchlorid, wahlweise in einer Menge von 1,0 mg/ml bis 6,0 mg/ml, optimalerweise von ungefähr 5,0 mg/ml, und/oder (ii) Glycyrrhizinsäure oder ein Salz davon, wahlweise in einer Menge von 0,8 mg/ml bis 1,2 mg/ml, optimalerweise von ungefähr 1,0 mg/ml, umfasst;
der Konservierungsstoff (i) Methyl-p-Hydroxybenzoat, wahlweise in einer Menge von 0,25 mg/ml bis 1,15 mg/ml, und/oder (ii) Propyl-p-Hydroxybenzoat, wahlweise in einer Menge von 0,03 mg/ml bis 0,15 mg/ml, und/oder (iii) Dinatrium-Kalzium-Edetat, wahlweise in einer Menge von 0,1 mg/ml bis 1,0 mg/ml, und/oder (iv) Natriumbenzoat, wahlweise in einer Menge von 0,2 mg/ml bis 5,0 mg/ml, umfasst.

8. Oromukosale Lösung gemäß einem der vorangehenden Ansprüche, welche ferner mindestens einen weiteren Bestandteil umfasst, der aus einem Viskositätsmittel, einem Süßungsmittel, einem Fluidisierungsmittel, einem Verdickungsmittel, einem Färbungsmittel und einer natürlichen Essenz eines Aromastoffs ausgewählt ist.

9. Oromukosale Lösung gemäß Anspruch 8, wobei der weitere Bestandteil aus Glycerol und/oder Sorbitol und/oder Xylitol und/oder Ethylalkohol und/oder Kastoröl-40-Polyethoxylat und/oder Saccharinnatrium und/oder Acesulfamkalium und/oder Minzeessenz und/oder natürlichem Minzearoma und/oder natürlichem Pfirsicharoma und/oder Patentblau V-E131, E-124 ausgewählt ist.

10. Oromukosale Lösung gemäß einem der Ansprüche 1 bis 8, welche ferner Xylitol umfasst.

11. Oromukosale Lösung gemäß einem der vorangehenden Ansprüche in der Form eines Mundwassers zum Sprühen, bevorzugt mit einem Dispensiervolumen von 100 Mikroliter (0,1 ml) bis 300 Mikroliter (0,3 ml), bevorzugt von ungefähr 200 Mikroliter (0,2 ml), für jede Dosierungseinheit.

12. Oromukosale Lösung gemäß einem der vorangehenden Ansprüche, wobei der Puffer D-Glukamin, Meglumin oder eine Mischung davon ist.

13. Verpackte oromukosale Lösung, die das in einem der Ansprüche 1 bis 12 definierte pharmazeutische Präparat enthält, ausgestattet mit einer Dosierpumpe.

14. Verfahren zur Herstellung des in einem der Ansprüche 1 bis 12 definierten pharmazeutischen Präparats, welches umfasst:
i) Auflösen eines Konservierungsstoffs/von Konservierungsstoffen in einer Lösung;
ii) Auflösen des Flurbiprofens in Wasser oder einer Wasser/Ethyl-Alkohol-Mischung und Puffern mit dem organischen Amin bis zum angegebenen pH-Wert;
iii) Zugeben beliebiger Hilfsstoffe zu der Lösung von Schritt i) und Vermengen der Lösung von Schritt i) mit der Lösung von Flurbiprofen und organischem Amin von Schritt ii);
iv) Ergänzung des Volumens (oder Gewichts) mit Wasser, falls notwendig, Angleichen des pH-Werts an den vorgeschriebenen Wert unter Zugabe von organischem Amin.

15. Verwendung einer oromukosalen Lösung zur Erzeugung eines entzündungshemmenden Mittels zur Behandlung des Munds, Rachens und/oder Zahnfleisches, wobei die pharmazeutische Zusammensetzung die Form einer wässrigen Lösung hat, welche umfasst:
das nichtsteroidale Antirheumatikum (NSAR) Flurbiprofen, welches analgetisch wirkt, in einer Menge von 1,5 mg/ml bis 8,0 mg/ml;
ein biologisch kompatibles pufferndes organisches Amin, das mit einer freien oder monosubstituierten Aminogruppe oder einer Mischung davon in einer zum Puffern des pH-Werts des Präparats in dem unten angegebenen Bereich geeigneten Menge vorgesehen ist;
einen pH-Wert in einem Bereich von 6,5 bis 8,0; und Reinstwasser; und
das biologisch kompatible puffernde organische Amin D-Glukamin, Meglumin, Trometamol (Tri-Puffer) oder eine Mischung davon ist.

## Revendications

1. Solution buccale, comprenant :
l'anti-inflammatoire non stéroïdien (AINS), flurbiprofène, qui présente une activité analgésique, en une quantité comprise entre 1,5 mg/ml et 8,0 mg/ml ;
un tampon compatible sur le plan biologique, composé essentiellement d'une amine organique sélectionnée parmi au moins l'un de la D-glucamine, de la méglumine, du trométamol (tampon tris) et d'un mélange de ceux-ci, en une quantité permettant de tamponner le pH de la préparation dans la plage spécifiée ci-dessous ;
un pH compris entre 6,5 et 8,0 ; et
de l'eau de qualité pharmaceutique.

2. Solution buccale selon la revendication 1, dans laquelle le flurbiprofène se trouve sous la forme d'un racémate ou d'un de ses énantiomères sélectionnés parmi R-(-)-flurbiprofène and S-(+)-flurbiprofène.

3. Solution buccale selon l'une quelconque des revendications 1 ou 2, comprenant du flurbiprofène en une quantité d'environ 2,5 mg/ml.

4. Solution buccale selon l'une quelconque des revendications précédentes, présentant un pH compris entre 7,0 et 7,5.

5. Solution buccale selon l'une quelconque des revendications précédentes, comprenant de la D-glucamine en une quantité comprise entre 0,35 mg/ml et 1,12 mg/ml ; de la méglumine en une quantité comprise entre 0,40 mg/ml et 2,4 mg/ml ; et/ou du trométamol en une quantité comprise entre 0,10 mg/ml et 0,75 mg/ml.

6. Solution buccale selon l'une quelconque des revendications précédentes, comprenant le tampon en une quantité permettant de tamponner le pH de la solution dans une plage comprise entre 7,0 et 7,5.

7. Solution buccale selon l'une quelconque des revendications précédentes, comprenant en outre :
un désinfectant léger ; et/ou
un ou plusieurs conservateurs ;
dans laquelle :
le désinfectant léger comprend au moins un parmi (i) le chlorure de cétylpyridinium, en une quantité éventuellement comprise entre 1,0 mg/ml et 6,0 mg/ml, en une quantité optimale d'environ 5,0 mg/ml, et (ii) l'acide glycyrrhizique ou un sel de celui-ci, en une quantité éventuellement comprise entre 0,8 mg/ml et 1,2 mg/ml, en une quantité optimale d'environ 1,0 mg/ml ;
le conservateur comprend au moins l'un parmi (i) le p-hydroxybenzoate de méthyle, en une quantité éventuellement comprise entre 0,25 mg/ml et 1,15 mg/ml, (ii) le p-hydroxybenzoate de propyle, en une quantité éventuellement comprise entre 0,03 mg/ml et 0,15 mg/ml, (iii) le calcitétracémate disodique, en une quantité éventuellement comprise entre 0,1 mg/ml et 1,0 mg/ml, et (iv) le benzoate de sodium, en une quantité éventuellement comprise entre 0,2 mg/ml et 5,0 mg/ml.

8. Solution buccale selon l'une quelconque des revendications précédentes, comprenant en outre au moins un ingrédient supplémentaire sélectionné parmi un agent de viscosité, un édulcorant, un agent fluidifiant, un épaississant, un colorant et une essence naturelle d'aromatisant.

9. Solution buccale selon la revendication 8, dans laquelle l'ingrédient supplémentaire est sélectionné parmi au moins l'un du glycérol, sorbitol, xylitol, éthanol, huile de ricin 40 éthoxylée, sodium de saccharine, acésulfame de potassium, essence de menthe, arôme naturel de menthe, arôme naturel de pêche et bleu patenté V-E131, E-124.

10. Solution buccale selon l'une quelconque des revendications 1 à 8, comprenant en outre du xylitol.

11. Solution buccale selon l'une quelconque des revendications précédentes sous forme de bain de bouche à pulvériser, présentant de préférence un volume de distribution pour chaque dose unitaire compris entre 100 microlitres (0,1 ml) et 300 micro litres (0,3 ml), de préférence d'environ 200 microlitres (0,2 ml).

12. Solution buccale selon l'une quelconque des revendications précédentes, dans laquelle le tampon est de la D-glucamine, de la méglumine ou un mélange des deux.

13. Solution buccale emballée contenant la préparation pharmaceutique définie par l'une quelconque des revendications 1 à 12, équipée d'une pompe doseuse.

14. Procédé de production de la préparation pharmaceutique définie par l'une quelconque des revendications 1 à 12, comprenant les étapes suivantes :
i) dissolution du ou des conservateurs dans une solution ;
ii) dissolution du flurbiproféne dans de l'eau ou un mélange eau/éthanol et tamponnage avec de l'amine organique au pH spécifié ;
iii) ajout de tout ingrédient auxiliaire à la solution de l'étape i), et mélange de la solution de l'étape i) à la solution de flurbiprofène et d'amine organique de l'étape ii) ;
iv) appoint du volume (ou poids) avec de l'eau, si nécessaire en ajustant le pH à la valeur prescrite par ajout d'amine organique.

15. Utilisation d'une solution buccale dans la fabrication d'un agent anti-inflammatoire pour le traitement de la bouche, de la gorge et/ou des gencives, dans laquelle la composition pharmaceutique se trouve sous la forme d'une solution aqueuse comprenant :
l'anti-inflammatoire non stéroïdien (AINS) flurbiprofène, qui présente une activité analgésique, en une quantité comprise entre 1,5 mg/ml et 8,0 mg/ml ;
une amine organique de tampon compatible sur le plan biologique, fournie avec un groupe amino libre ou monosubstitué, ou un mélange de ceux-ci, en une quantité permettant de tamponner le pH de la préparation dans la plage spécifiée ci-dessous ;
un pH compris entre 6,5 et 8,0; et
de l'eau de qualité pharmaceutique ; et
l'amine organique de tampon compatible sur le plan biologique est la D-glucamine, la méglumine, le trométamol (tampon tris) ou un mélange de ceux-ci.
